(19) Europäisches Patentamt
European Patent Office
Office européen des brevets

(11) **EP 4 379 020 A1**

(12) **EUROPEAN PATENT APPLICATION**

(43) Date of publication:
**05.06.2024 Bulletin 2024/23**

(21) Application number: **23211354.8**

(22) Date of filing: **22.11.2023**

(51) International Patent Classification (IPC):
**C10G 1/00** (2006.01)    **C10G 11/05** (2006.01)
**C10G 11/18** (2006.01)    **B01J 29/40** (2006.01)
**B01J 37/00** (2006.01)

(52) Cooperative Patent Classification (CPC):
**C10G 1/002; B01J 29/40; B01J 29/405;**
**B01J 37/0045; C10G 11/05; C10G 11/18;**
B01J 2229/42; C10G 1/10; C10G 2300/1003;
C10G 2400/20

(84) Designated Contracting States:
**AL AT BE BG CH CY CZ DE DK EE ES FI FR GB**
**GR HR HU IE IS IT LI LT LU LV MC ME MK MT NL**
**NO PL PT RO RS SE SI SK SM TR**
Designated Extension States:
**BA**
Designated Validation States:
**KH MA MD TN**

(30) Priority: **01.12.2022 KR 20220165399**

(71) Applicants:
• **SK Innovation Co., Ltd.**
**Seoul 03188 (KR)**
• **SK Geo Centric Co., Ltd.**
**Jongno-gu**
**Seoul 03161 (KR)**

(72) Inventors:
• **JEON, Hee Jung**
**34124 Daejeon (KR)**
• **KIM, Ok Youn**
**34124 Daejeon (KR)**
• **LEE, Ho Won**
**34124 Daejeon (KR)**

(74) Representative: **Frick, Robert**
**Lorenz Seidler Gossel**
**Rechtsanwälte Patentanwälte**
**Partnerschaft mbB**
**Widenmayerstraße 23**
**80538 München (DE)**

(54) **METHOD AND DEVICE FOR CONVERTING WASTE PLASTIC PYROLYSIS OIL INTO LIGHT OLEFINS WITH HIGH YIELD**

(57) The present invention relates to a method for converting waste plastic pyrolysis oil into light olefins with a high yield, the method including: (1) putting waste plastic pyrolysis oil into a reactor; (2) allowing the waste plastic pyrolysis oil to react in the reactor in the presence of a catalytic cracking catalyst containing a compound of an alkali metal or an alkaline earth metal and a zeolite; and (3) recovering light olefins by separating the catalytic cracking catalyst and oil from a product obtained in the step (2).

EP 4 379 020 A1

**Description**

**TECHNICAL FIELD**

The present invention relates to a method and device for converting waste plastic pyrolysis oil into light olefins with a high yield.

**BACKGROUND**

Waste plastics, which are produced using petroleum as a feedstock, are difficult to recycle and are mostly disposed of as garbage. These wastes take a long time to degrade in nature, which causes contamination of the soil and serious environmental pollution. As a method for recycling waste plastics, there is a method for pyrolyzing and converting waste plastics into usable oil, and the obtained oil is called waste plastic pyrolysis oil.

Since waste plastic pyrolysis oil is a mixture of hydrocarbon oils having various boiling points and various molecular weight distributions, and a composition or reaction activity of impurities in the pyrolysis oil varies depending on the boiling point and molecular weight distribution characteristics of the mixture of hydrocarbon oils, the waste plastic pyrolysis oil cannot be directly used in the petrochemical industry or in the field, and needs to go through a separation process by boiling point or a refinery process.

In the mixture of hydrocarbon oils, olefins, for example, light olefins such as ethylene and propylene, have been widely used in the petrochemical industry. Until now, most ethylene or propylene has been mainly produced by pyrolysis of hydrocarbon oils containing a paraffinic compound as a main component, such as natural gas, naphtha oil, and gas oil in a steam atmosphere at a high temperature of 800°C or higher under a catalyst-free condition, but the method described above has problems of a low conversion rate of hydrocarbons and low olefin selectivity. As a measure to improve reaction efficiency such as a conversion rate of hydrocarbons or olefin selectivity, a fluid catalytic cracking (FCC) process has been performed. Representative examples of the FCC process include a catalytic cracking process using an acid catalyst. Among various acid catalysts, zeolites have been most widely used, and as representative zeolites for catalytic cracking, ZSM-5, USY, REY, β-zeolite, and the like have been used.

However, waste plastic pyrolysis oil contains an excessive amount of impurities such as chlorine, nitrogen, or a metal compared to crude oil, natural gas, and naphtha oil, and in the fluid catalytic cracking process of waste plastic pyrolysis oil, a catalytic active site is neutralized or deactivated by the impurities, and thus the reaction activity is significantly reduced. In the case of waste plastic pyrolysis oil according to the related art, a conversion process has been performed as a method of removing a deactivated waste catalyst in the middle of the process and making-up a new catalyst again, but since the deactivated catalyst contains an excessive amount of impurities and its deactivated amount is significant, serious environmental problems are caused, and there are economic and environmental constraints on its disposal and treatment. In addition, in the process of removing a waste catalyst and making-up a new catalyst, as a heat loss or energy loss occurs, process efficiency is significantly reduced and it is difficult to maintain uniform reaction activity, and as a result, constant quality may not be obtained. The most serious problem is that the conversion yield from waste plastic pyrolysis oil into light olefins is remarkably low, and impurities such as chlorine, nitrogen, or a metal are still contained in the converted light olefins, which makes it difficult to economically and commercially put the converted light olefins into practical use due to deterioration of quality.

Therefore, there is a demand for a technology capable of producing light olefins from waste plastic pyrolysis oil with a high yield in a fluid catalytic cracking process.

**SUMMARY**

The present invention aims to providing a method and device for converting waste plastic pyrolysis oil into light olefins with a high yield.

The present invention further aims to providing a method and device for converting waste plastic pyrolysis oil into light olefins with a high yield that may implement a conversion yield from waste plastic pyrolysis oil into light olefins of 10% or more, specifically, 25% or more, and more specifically, 40% or more.

The present invention yet further aims to providing a method and device for converting waste plastic pyrolysis oil into light olefins with a high yield that may stably perform a light olefin conversion process for a long time by preventing catalytic deactivation due to impurities contained in the waste plastic pyrolysis oil.

The present invention also aims to providing a method and device for converting waste plastic pyrolysis oil into light olefins with a high yield that may minimize a content of impurities in light olefins converted from waste plastic pyrolysis oil.

Against this background, the present invention relates to a method for converting waste plastic pyrolysis oil into light olefins, the method including: (1) putting waste plastic pyrolysis oil into a reactor; (2) allowing the waste plastic pyrolysis oil to react in the reactor in the presence of a catalytic cracking catalyst containing a compound of an alkali metal or an alkaline earth metal and a zeolite; and (3) recovering light olefins by separating the catalytic cracking catalyst and oil from a product obtained in the step (2).

In an exemplary embodiment, the compound of an alkali metal or an alkaline earth metal may include an oxide, a hydroxide, a carbonate, a silicate, a sulfate, an acetate, a nitride, or an alkoxide.

In an exemplary embodiment, the compound of an alkali metal or an alkaline earth metal may include magnesium oxide.

In an exemplary embodiment, the catalytic cracking catalyst may satisfy an Mg/Al molar ratio of 10 to 40.

In an exemplary embodiment, the zeolite may include ZSM-5, ZSM-11, Y-zeolite, ferrierite, mordenite, MCM-22, SUZ-4, or L-type zeolite.

In an exemplary embodiment, the catalytic cracking catalyst may further contain clay and a binder.

In an exemplary embodiment, the binder may contain a silica-based compound or an aluminum-based compound.

In an exemplary embodiment, the catalytic cracking catalyst may contain 1 to 10 wt% of the compound of an alkali metal or an alkaline earth metal, 20 to 70 wt% of the zeolite, 10 to 60 wt% of the clay, and 10 to 50 wt% of the binder.

In an exemplary embodiment, the catalytic cracking catalyst may have an average particle size of 50 to 2,000 pm.

In an exemplary embodiment, the reactor may include a fluidized bed reactor.

In an exemplary embodiment, the waste plastic pyrolysis oil may contain a vacuum gas oil (VGO) component having a boiling point of 340°C or higher at atmospheric pressure.

In an exemplary embodiment, the step (2) may be performed at a temperature of 400 to 600°C and a reaction pressure of 50 to 200 kPa.

In an exemplary embodiment, the method for converting waste plastic pyrolysis oil into light olefins with a high yield may further include: (3-1) recovering the catalytic cracking catalyst separated in the step (3), putting the recovered catalytic cracking catalyst into a regenerator, and then oxidizing the compound of an alkali metal or an alkaline earth metal; and (3-2) circulating the catalytic cracking catalyst containing the oxidized compound of an alkali metal or an alkaline earth metal to the step (2) and re-feeding the catalytic cracking catalyst containing the oxidized compound of an alkali metal or an alkaline earth metal into the reactor.

In an exemplary embodiment, the step (3-1) may be performed at 600 to 800°C in the presence of oxygen gas.

In an exemplary embodiment, the method for converting waste plastic pyrolysis oil into light olefins with a high yield may further include, before the step (3-1), stripping the catalytic cracking catalyst separated in the step (3).

In an exemplary embodiment, in the recovered light olefins, a content of chlorine may be 10 ppm or less and a content of nitrogen may be 50 ppm or less.

The invention further relates to a device for converting waste plastic pyrolysis oil into light olefins, the device including: a fluidized bed reactor configured to receive waste plastic pyrolysis oil and to perform a catalytic cracking reaction is performed in the presence of a catalytic cracking catalyst, the catalytic cracking catalyst containing a compound of an alkali metal or an alkaline earth metal and a zeolite; a cyclone configured to receive a product from the fluidized bed reactor and to separate the product into the catalytic cracking catalyst and oil; and a stabilizer configured to receive the oil from the cyclone and to separate the oil into a gas component and a liquid component.

The device is preferably configured to carry out a method of the invention.

In an exemplary embodiment, the compound of an alkali metal or an alkaline earth metal may include an oxide, a hydroxide, a carbonate, a silicate, a sulfate, an acetate, a nitride, or an alkoxide.

In an exemplary embodiment, the compound of an alkali metal or an alkaline earth metal may include magnesium oxide.

In an exemplary embodiment, the device may further include: a regenerator configured to receive the catalytic cracking catalyst from the cyclone and to oxidize the compound of an alkali metal or an alkaline earth metal, and a recirculation line configured to re-introduce the catalytic cracking catalyst containing the oxidized compound of an alkali metal or an alkaline earth metal into the fluidized bed reactor from the regenerator.

Other features and aspects will be apparent from the following detailed description, the drawings, and the claims.

## DETAILED DESCRIPTION OF EMBODIMENTS

Unless otherwise defined, a unit of "%" used in the present specification refers to "wt%".

Unless otherwise defined, a "boiling point" used in the present specification is based on atmospheric pressure, and the expression "bp" or the like refers to a boiling point.

Unless otherwise defined, a unit of "ppm" used in the present specification refers to "mass ppm".

Waste plastic pyrolysis oil is a mixture of hydrocarbon oils produced by pyrolysis of waste plastics, and the waste plastics may be solid or liquid wastes related to synthetic polymer compounds such as waste synthetic resins, waste synthetic fibers, waste synthetic rubber, and waste vinyl. The mixture of hydrocarbon oils may contain impurities such as a chlorine compound, a nitrogen compound, or a compound of an alkali metal or an alkaline earth metal, in addition to the hydrocarbon oils, may contain impurities in the form of compounds in which chlorine, nitrogen, or a metal is bonded to hydrocarbons, and may contain hydrocarbons in the form of olefins. For example, the waste plastic pyrolysis oil may contain 300 ppm or more of nitrogen, 30 ppm or more of chlorine, 30 ppm or more of a metal, 20 vol% or more of an olefin, and 1 vol% or more of a conjugated diolefin. As described above, when a fluid catalytic cracking process is performed on waste plastic pyrolysis oil containing various impurities by a method according to the related art, reaction activity and reaction yield are significantly reduced due to the impurities, and there is a fatal problem that impurities such as chorine, nitrogen, or a metal are still present in the converted light olefins.

Accordingly, according to a method of the present invention, a conversion yield of light olefins from waste plastic pyrolysis oil may be significantly improved, a light olefin conversion process may be stably performed for a long time by suppressing catalytic deactivation due to impurities, and a content of impurities such as chlorine or nitrogen in the converted light olefins may be minimized. That is, the problems of both low yield and impurity content occurring when a feedstock is waste plastic pyrolysis oil may be solved, and high-quality light olefins with minimized impurities may be obtained from the waste plastic pyrolysis oil with a high yield.

Specifically, the present invention provides a method for converting waste plastic pyrolysis oil into light olefins, the method including: (1) putting waste plastic pyrolysis oil into a reactor; (2) allowing the waste plastic pyrolysis oil to react in the reactor in the presence of a catalytic cracking catalyst containing a compound of an alkali metal or an alkaline earth metal and a zeolite; and (3) recovering light olefins by separating the catalytic cracking catalyst and oil from a product obtained in the step (2).

The waste plastic pyrolysis oil includes a mixture of hydrocarbon oils, and includes, for example, a mixture of hydrocarbon oils having various boiling points and molecular weight distributions such as C7 to C9 Naphtha having a boiling point of 80 to 150°C at atmospheric pressure, C10 to C17 Kerosene having a boiling point of 150 to 265°C, C18 to C20 LGO having a boiling point of 265 to 340°C, and C21- VGO/AR having a boiling point of 340°C or higher. As described below, VGO has conversion efficiency that is much lower than those of Kerosene and Naphtha in a light olefin conversion process according to the related art, making it difficult to use VGO, and therefore, the conversion process has been performed mainly using Kerosene and Naphtha as a feedstock excluding VGO. On the other hand, the light olefin high-yield conversion process of the present invention may implement a significantly excellent cracking efficiency and light olefin conversion efficiency even when waste plastic pyrolysis oil containing a vacuum gas oil (VGO) component is used as a feedstock.

The waste plastic pyrolysis oil including a mixture of various hydrocarbon oils may be converted into light olefins with a high yield using the catalytic cracking catalyst containing a compound of an alkali metal or an alkaline earth metal and a zeolite. In addition, the compound of an alkali metal or an alkaline earth metal may prevent deactivation of the catalytic cracking catalyst by trapping impurities contained in the waste plastic pyrolysis oil, such as chlorine, nitrogen, sulfur, or oxygen, and may minimize impurities in the converted light olefins.

In an exemplary embodiment, the alkali metal may include a metal selected from lithium, sodium, potassium, and rubidium, and the alkaline earth metal may include a metal selected from magnesium, calcium, strontium, and barium. Specifically, considering the ability to trap impurities, the alkali metal may be sodium or potassium, and the alkaline earth metal may be magnesium or calcium.

In an exemplary embodiment, the compound of an alkali metal or an alkaline earth metal may include an oxide, a hydroxide, a carbonate, a silicate, an acetate, a nitride, or an alkoxide of an alkali metal or an alkaline earth metal. As another exemplary embodiment, the compound of an alkali metal or an alkaline earth metal may have a structure. For example, the compound of an alkali metal or an alkaline earth metal may have a hydrotalcite-like structure, a double-layer hydroxide structure, a spinel structure, or a perovskite structure. The compound of an alkali metal or an alkaline earth metal described above may prevent deactivation of the catalytic cracking catalyst by trapping impurities contained in the waste plastic pyrolysis oil, such as chlorine, nitrogen, sulfur, or oxygen, and may minimize impurities in the converted light olefins.

Preferably, considering the light olefin conversion efficiency and impurity removal efficiency of the waste plastic pyrolysis oil, the compound of an alkali metal or an alkaline earth metal may be preferably potassium oxide, calcium oxide, or magnesium oxide, and may be most preferably magnesium oxide. The magnesium oxide has the best ability to trap chlorine among the impurities, such that the magnesium oxide may effectively remove chlorine impurities contained in the waste plastic pyrolysis oil.

In an exemplary embodiment, the zeolite may include ZSM-5, ZSM-11, Y-zeolite, ferrierite, mordenite, MCM-22, SUZ-4, or L-type zeolite. A zeolite is a porous molecular sieve having a rich pore structure and a large specific surface area, and has a lot of active sites and excellent catalytic cracking efficiency. The zeolite may control the cracking activity according to the number of atoms constituting the pores, a pore size, or a stereostructure (one-dimensional, two-dimensional, or three-dimensional structure) of the pores. Preferred zeolites include ZSM-5 or Y-zeolite.

In an exemplary embodiment, the catalytic cracking catalyst may further contain clay and a binder. As the catalytic cracking catalyst further contains a zeolite, clay, and a binder, the catalytic cracking catalyst may have high mechanical strength and may be used in a large-scale petrochemical process such as a process of upgrading waste plastic pyrolysis oil.

The binder may contain a silica-based compound or an aluminum-based compound. For example, the binder may include silica sol, a silicic acid solution, water glass, alumina, alumina-silica, and pseudo-boehmite alumina (PBA).

The clay may comprise kaolin or a mixture of kaolin and montmorillonite. In this case, Clay has a characteristic of having a weak acid site and can crack the heavy hydrocarbon at high temperatures. The weak acid site means an acid site at which ammonia can be desorbed at 400 °C or less at ammonia TPD analysis. Also, the clay used is good when the surface area is large and the mesopores are well developed, but the clay of low surface area does not mean that it cannot be used.

In an exemplary embodiment, the catalytic cracking catalyst may contain 1 to 10 wt% of the compound of an alkali metal or an alkaline earth metal, 20 to 70 wt% of the zeolite, 10 to 60 wt% of the clay, and 10 to 50 wt% of the binder. When the catalytic cracking catalyst satisfies all the above weight ranges, the light olefin conversion yield may be significantly improved, and the catalytic deactivation may be effectively prevented by adsorption of the impurities, and as a result, the fluid catalytic cracking process may be stably performed for a long time.

Each component contained in the catalytic cracking catalyst will be individually described. When the compound of an alkali metal or an alkaline earth metal is contained in the above weight range, the light olefin conversion yield and the impurity removal effect may be improved, and the activity of the catalytic cracking catalyst and hydrothermal stability may also be improved. Specifically, the compound of an alkali metal or an alkaline earth metal may be contained in an amount of 1 to 8 wt%, and more specifically, may be contained in an amount of 3 to 6 wt%.

When the zeolite is contained in the weight range, the catalytic cracking efficiency may be improved, and the light olefin conversion yield may be maximized. Specifically, the zeolite may be contained in an amount of 20 to 60 wt%, and more specifically, may be contained in an amount of 30 to 50 wt%.

When the clay is contained in the above weight range, the specific gravity of the catalytic cracking catalyst and the overall activity of the catalytic cracking catalyst may be optimized. Specifically, the clay may be contained in an amount of 10 to 50 wt%, and more specifically, may be contained in an amount of 20 to 40 wt%.

When the binder is contained in the above weight range, physical properties such as abrasion strength of the catalytic cracking catalyst may be excellently maintained. Specifically, the binder may be contained in an amount of 10 to 40 wt%, and more specifically, may be contained in an amount of 15 to 35 wt%.

In an exemplary embodiment, the catalytic cracking catalyst may satisfy an Mg/Al molar ratio of 10 to 40. As described above, considering the light olefin conversion efficiency and the impurity removal efficiency, the compound of an alkali metal or an alkaline earth metal may be most preferably magnesium oxide. The magnesium oxide adsorbs impurities such as chlorine, nitrogen, or sulfur contained in the waste plastic pyrolysis oil, such that it is possible to prevent a solid acid active site in the zeolite from being deactivated. However, when a content of the magnesium oxide is too high, the activation of zeolite may be interfered with, such that the cracking activity and the light olefin conversion yield may be rather reduced, and when the content of the magnesium oxide is too low, the impurity removal effect may be insufficient. Therefore, when the Mg/Al molar ratio satisfies 10 to 40 by optimizing the molar ratio of Mg in the magnesium oxide to Al in the zeolite, the impurity removal effect and the light olefin conversion yield may be maximized. Preferably, the Mg/Al molar ratio may be 10 to 35, and more preferably, the Mg/Al molar ratio may be 15 to 30.

In an exemplary embodiment, the catalytic cracking catalyst may have an average particle size of 50 to 2,000 pm. In a case where a catalytic cracking catalyst having the above size is screened and used to correspond to intrinsic properties of waste plastic pyrolysis oil, such as a viscosity and a density, the olefin selectivity and the catalytic activity may be significantly improved. Specifically, the average particle size may be 50 to 1,000 pm, and more specifically, may be 50 to 700 pm. More specifically, the average particle size may be preferably 50 to 200 pm, and most specifically, may be 80 to 150 pm.

In an exemplary embodiment, the catalytic cracking catalyst may have a total specific surface area of 50 to 150 $m^2$/g and an apparent density of 0.5 to 1 g/$cm^3$. Within the above ranges, a contact area with the feedstock and the cracking efficiency are optimized, such that the light olefin conversion yield may be improved. Specifically, the total specific surface area may be 50 to 130 $m^2$/g and the apparent density may be 0.5 to 0.8 g/$cm^3$, and more specifically, the total specific surface area may be 700 to 100 $m^2$/g and the apparent density may be 0.6 to 0.7 g/$cm^3$.

In an exemplary embodiment, the catalytic cracking catalyst may satisfy the following Expression 1.

[Expression 1]

$$2 < (D90-D10)/D50 < 5$$

Expression 1 represents a particle size distribution width of the catalytic cracking catalyst in a volume-based distribution measured with a laser diffraction particle size distribution measuring device, D10 is a 10% cumulative diameter, D50 is a 50% cumulative diameter, and D90 is a 90% cumulative diameter.

As the particle size distribution width of the catalytic cracking catalyst satisfies Expression 1, a structural collapse of the zeolite due to steam may be prevented, such that catalytic stability may be improved. As described below, the catalytic cracking catalyst is used in both a reactor process in a riser of a fluidized bed reactor and a regeneration process in a regenerator, such that the stability and the catalytic cracking efficiency of the catalytic cracking catalyst may be more excellent. Specifically, Expression 1 may be 2 < (D90-D10)/D50 < 4, and more specifically, Expression 1 may be 2 < (D90-D10)/D50 < 3.

The method for converting waste plastic pyrolysis oil into light olefins of the present invention will be more specifically described with reference to embodiments in the following.

In the step (1) of putting the waste plastic pyrolysis oil into the reactor, the reactor may be a fluidized bed reactor. In a case where a fixed bed reactor is used, although a yield of olefins is high at the beginning of the reaction in which hydrocarbons are in contact with the catalytic cracking catalyst, deactivation of the catalyst and excessive production of coke occur over time, such that a conversion rate of hydrocarbons and the yield of olefins are reduced as a whole, and a lot of energy is consumed in a regeneration process. Using a fluidized bed reactor may be preferable in terms of a catalyst regeneration process described below as well as solving the above problems. In addition, improved light olefin conversion efficiency may be realized. In this case, the fluidized bed reactor may be a riser.

In an exemplary embodiment, the waste plastic pyrolysis oil may contain a vacuum gas oil (VGO) component having a boiling point of 340°C or higher at atmospheric pressure. In the related art, VGO has conversion efficiency that is much lower than those of Kerosene and Naphtha in the light olefin conversion process, making it difficult to use VGO, and therefore, the conversion process has been performed mainly using Kerosene and Naphtha as a feedstock excluding VGO. On the other hand, the light olefin high-yield conversion process of the present invention may implement an excellent cracking efficiency and light olefin conversion efficiency even when waste plastic pyrolysis oil containing a vacuum gas oil (VGO) component is used as a feedstock.

The step (2) of allowing the waste plastic pyrolysis oil to react in the reactor in the presence of the catalytic cracking catalyst containing a compound of an alkali metal or an alkaline earth metal and a zeolite may be performed at a temperature of 400 to 600°C and a reaction pressure of 50 to 200 kPa. Since the reaction efficiency in the step (2) is highly dependent on the temperature and pressure, the energy consumption may be minimized under the above conditions, and the catalytic deactivation may be effectively suppressed. Specifically, the temperature may be 400 to 550°C and the pressure may be 50 to 150 kPa, and more specifically, the temperature may be 400 to 500°C and the pressure may be 50 to 100 kPa.

In addition, the reaction efficiency in the step (2) may be dependent on a plateau time, a catalyst/pyrolysis oil ratio, or a pyrolysis oil/steam ratio. The plateau time may be about 0.1 to 600 seconds, the catalyst/pyrolysis oil ratio may be 1 to 50, and the pyrolysis oil/steam ratio may be 0.01 to 10, specifically, the plateau time may be about 0.5 to 120 seconds, the catalyst/pyrolysis oil ratio may be 5 to 30, and the pyrolysis oil/steam ratio may be 0.1 to 2.0, and more specifically, the plateau time may be about 1 to 20 seconds, the catalyst/pyrolysis oil ratio may be 10 to 20, and the pyrolysis oil/steam ratio may be 0.3 to 1.

After performing the catalytic cracking reaction, light olefins may be recovered by separating a reaction product into the catalytic cracking catalyst and oil through the step (3) . Specifically, a reaction product obtained in the step (2) may be introduced into a cyclone described below, the catalytic cracking catalyst and oil may be separated within a short time, and light olefins may be recovered from the oil.

In an exemplary embodiment, the method for converting waste plastic pyrolysis oil into light olefins with a high yield may further include: (3-1) recovering the catalytic cracking catalyst separated in the step (3), putting the recovered catalytic cracking catalyst into a regenerator, and then oxidizing the compound of an alkali metal or an alkaline earth metal; and (3-2) circulating the catalytic cracking catalyst containing the oxidized compound of an alkali metal or an alkaline earth metal to the step (2) and re-feeding the catalytic cracking catalyst containing the oxidized compound of an alkali metal or an alkaline earth metal into the reactor. As the separated catalytic cracking catalyst is put into the regenerator, the compound of an alkali metal or an alkaline earth metal is oxidized in the presence of oxygen gas, the catalytic cracking catalyst containing the oxidized compound of an alkali metal or an alkaline earth metal is circulated to the step (2) again, and the catalytic cracking catalyst containing the oxidized compound of an alkali metal or an alkaline earth metal is re-fed into the reactor, economic feasibility may be improved, and impurities such as chlorine or nitrogen contained in the waste plastic pyrolysis oil may be removed. Specifically, the reactor may be a riser of a fluidized bed reactor, and the regenerator may be a regenerator of a fluidized bed reactor. The impurities are bound to and decomposed by the catalytic cracking catalyst containing the compound of an alkali metal or an alkaline earth metal, and may be continuously discharged in a gaseous state in the process, thereby removing the impurities. In addition, the method for converting waste plastic pyrolysis oil into light olefins with a high yield further includes the step (3-1) and the step (3-2), such that a heat loss or energy loss generated in a process of removing a waste catalyst and making-up a new catalytic cracking catalyst in the related art may be solved.

In an exemplary embodiment, the step (3-1) may be performed at 600 to 800°C in the presence of oxygen gas. The oxidation process may be performed by additionally injecting oxygen gas into the regenerator, and the reaction efficiency may be the most excellent in the above temperature range. Specifically, the temperature may be 600 to 750°C, and more specifically, may be 600 to 700°C.

The process of adsorbing and decomposing the impurities contained in the waste plastic pyrolysis oil by the compound of an alkali metal or an alkaline earth metal contained in the catalytic cracking catalyst in the light olefin conversion process according to an exemplary embodiment of the present invention will be specifically described. For example, when the compound of an alkali metal or an alkaline earth metal is magnesium oxide, chorine (Cl) impurities may be adsorbed and decomposed by the magnesium oxide through reactions such as the following Reaction Formulas 1 and 2.

[Reaction Formula 1] $\quad MgO + 2HCl \rightarrow MgCl_2 + H_2$

[Reaction Formula 2] $\quad 2MgCl_2 + O_2 \rightarrow 2MgO + 2Cl_2$

Nitrogen impurities may be adsorbed and decomposed by magnesium oxide through the following Reaction Formulas 3 and 4.

[Reaction Formula 3] $\quad MgO + NO_2 \rightarrow MgNO_3$

[Reaction Formula 4] $\quad 2MgNO_3 + 7H_2 \rightarrow 2MgO + 2NH_3 + 4H_2O$

Sulfur impurities may be adsorbed and decomposed by magnesium oxide through the following Reaction Formulas 5 to 8.

[Reaction Formula 5] $\quad MgO + SO_2 + 1/2O_2 \rightarrow MgSO_4$

[Reaction Formula 6] $\quad MgSO_4 + 4H_2 \rightarrow MgO + H_2S + 3H_2O$

[Reaction Formula 7] $\quad MgSO_4 + 4H_2 \rightarrow MgS + 4H_2O$

[Reaction Formula 8] $\quad MgS + H_2O \rightarrow MgO + H_2S$

In an exemplary embodiment, the method for converting waste plastic pyrolysis oil into light olefins with a high yield may further include, before the step (3-1), stripping the catalytic cracking catalyst separated in the step (3) . Since the catalytic cracking catalyst separated in the step (3) may contain unseparated hydrocarbon oils, the method further includes the stripping step for removing the unseparated hydrocarbon oils, such that the regeneration efficiency may be improved.

As an exemplary embodiment, the catalytic cracking catalyst may be a composite catalyst obtained by mixing a compound of an alkali metal or an alkaline earth metal, a zeolite, clay, and a binder, and then performing molding. Such a composite catalyst may realize an excellent impurity removal effect by modifying the entire catalytic cracking catalyst with the compound of an alkali metal or an alkaline earth metal.

As another exemplary embodiment, the catalytic cracking catalyst may be a binary composite catalyst in which a molded catalyst containing a zeolite, clay, and a binder, a molded catalyst of a compound of an alkali metal or an alkaline earth metal, and a structure thereof are mixed. As described above, the compound of an alkali metal or an alkaline earth metal may have a structure. For example, the compound of an alkali metal or an alkaline earth metal may have a hydrotalcite-like structure, a double-layer hydroxide structure, a spinel structure, or a perovskite structure. Such a binary composite catalyst has excellent regeneration efficiency and may maintain catalytic activity for a long time.

As an exemplary embodiment, a method for preparing a catalytic cracking catalyst may be as follows. The method for preparing a catalytic cracking catalyst may include: preparing a mixed solid fine powder by mixing and crushing a zeolite, clay, and a compound of an alkali metal or an alkaline earth metal; preparing a binder containing alumina gel; preparing a mixed slurry by uniformly mixing the mixed solid fine powder and the binder; preparing a spherical catalyst by spray-drying the mixed slurry and then performing calcination; recovering a catalyst having an average particle size of 5 to 200 $\mu$m through sieving; and ion-exchanging the recovered catalyst with an aqueous solution of rare earth metal (RE) chloride containing cerium and lanthanum and then performing calcination. The spherical molded catalyst prepared by such a method has an excellent effect of adsorbing impurities by the compound of an alkali metal or an alkaline earth metal and excellent hydrothermal stability, may effectively protect the acid site of the zeolite, and above all, may convert waste plastic pyrolysis oil into light olefins with a relatively high yield during catalytic cracking. In addition, the catalytic cracking catalyst may exhibit high cracking activity and stability even in a high temperature and high humidity atmosphere.

In an exemplary embodiment, a yield of converting the waste plastic pyrolysis oil into light olefins may be 10% or more. Light olefins may be converted from waste plastic pyrolysis oil with high efficiency using the catalytic cracking catalyst containing a compound of an alkali metal or an alkaline earth metal and a zeolite. Specifically, a conversion yield into light olefins may be 25% or more, and more specifically, the conversion yield into light olefins may be 40% or more. The conversion yield into light olefins may be 90% or less.

In an exemplary embodiment, in the light olefins converted from the waste plastic pyrolysis oil, a content of chlorine may be 10 ppm or less and a content of nitrogen may be 50 ppm or less. The process is performed using the catalytic cracking catalyst containing a compound of an alkali metal or an alkaline earth metal and a zeolite, such that light olefins with minimized impurities may be obtained. The compound of an alkali metal or an alkaline earth metal may effectively remove chlorine because it has the most excellent ability to trap chlorine among the impurities. In addition, the compound of an alkali metal or an alkaline earth metal may also reduce impurities such as nitrogen or sulfur, and specifically, in the converted light olefins, the content of chlorine and the content of nitrogen may be 5 ppm or less and 30 ppm or less, respectively, and more specifically, the content of chlorine and the content of nitrogen may be 3 ppm or less and 10 ppm or less, respectively. The content of chlorine and the content of nitrogen may be 0.01 ppm or more and 0.1 ppm or more, respectively.

In addition, the present invention provides a device for converting waste plastic pyrolysis oil into light olefins, the device including: a fluidized bed reactor into which waste plastic pyrolysis oil is introduced and in which a catalytic cracking reaction is performed in the presence of a catalytic cracking catalyst containing a compound of an alkali metal or an alkaline earth metal and a zeolite; a cyclone into which a product is introduced from the fluidized bed reactor and in which the product is separated into the catalytic cracking catalyst and oil; and a stabilizer into which the oil is introduced from the cyclone and in which the oil is separated into a gas component and a liquid component.

In an exemplary embodiment, the alkali metal may include a metal selected from lithium, sodium, potassium, and rubidium, and the alkaline earth metal may include a metal selected from magnesium, calcium, strontium, and barium. Specifically, considering the ability to trap impurities, the alkali metal may be sodium or potassium, and the alkaline earth metal may be magnesium or calcium.

In an exemplary embodiment, the compound of an alkali metal or an alkaline earth metal may include an oxide, a hydroxide, a carbonate, a silicate, an acetate, a nitride, or an alkoxide of an alkali metal or an alkaline earth metal. As another exemplary embodiment, the compound of an alkali metal or an alkaline earth metal may have a structure. For example, the compound of an alkali metal or an alkaline earth metal may have a hydrotalcite-like structure, a double-layer hydroxide structure, a spinel structure, or a perovskite structure. The compound of an alkali metal or an alkaline earth metal described above may prevent deactivation of the catalytic cracking catalyst by trapping impurities contained in the waste plastic pyrolysis oil, such as chlorine, nitrogen, sulfur, or oxygen, and may minimize impurities in the converted light olefins.

Preferably, considering the light olefin conversion efficiency and impurity removal efficiency of the waste plastic pyrolysis oil, the compound of an alkali metal or an alkaline earth metal may be preferably potassium oxide, calcium oxide, or magnesium oxide, and may be most preferably magnesium oxide. The magnesium oxide has the best ability to trap chlorine among the impurities, such that the magnesium oxide may most effectively remove chlorine.

The waste plastic pyrolysis oil is fed into the fluidized bed reactor, and may be fed after being heated to a temperature of 30 to 600°C for a smoother reaction. The waste plastic pyrolysis oil may be mixed with the catalytic cracking catalyst containing a compound of an alkali metal or an alkaline earth metal and a zeolite provided in a fluidized bed reactor, or may be mixed with the catalytic cracking catalyst to be fed from the regenerator through a recirculation line connected to a fluidized bed reactor.

In the fluidized bed reactor, a catalytic cracking reaction may be performed at a temperature of 400 to 600°C and a pressure of 50 to 200 kPa, and in this case, the fluidized bed reactor may be a riser. Therefore, the product may be introduced into the cyclone and may be separated into oil and the catalytic cracking catalyst. The separated oil is introduced into the stabilizer from the cyclone, and may be separated into a gas component and a liquid component through cooling.

In an exemplary embodiment, the device for converting waste plastic pyrolysis oil into light olefins with a high yield may further include a regenerator into which the catalytic cracking catalyst is introduced from the cyclone and in which the compound of an alkali metal or an alkaline earth metal is oxidized, and the catalytic cracking catalyst containing the oxidized compound of an alkali metal or an alkaline earth metal may be re-introduced into the fluidized bed reactor from the regenerator through a recirculation line. The separated catalytic cracking catalyst may be accumulated in the cyclone, and a stripping steam may be fed through a separate line connected to the bottom of the cyclone. The stripping steam removes and discharges an unseparated hydrocarbon reaction product contained in the catalytic cracking catalyst while moving upward along the cyclone. The catalytic cracking catalyst that has passed through the stripping steam in the cyclone is introduced into the regenerator, and in this case, impurities such as chlorine or nitrogen contained in the waste plastic pyrolysis oil are adsorbed to the compound of an alkali metal or an alkaline earth metal contained in the catalytic cracking catalyst. Gas such as air containing oxygen is introduced into the regenerator through a separate inlet, the catalytic cracking catalyst and oxygen react with each other at a high temperature of 600°C or higher, and as a result, the impurities adsorbed to the compound of an alkali metal or an alkaline earth metal may be removed by being discharged as a gas. The catalytic cracking catalyst treated as described above may be re-introduced into the reactor through the recirculation line.

Hereinafter, the present invention will be described in detail with reference to Examples.

**Example 1**

1) Feedstock

200 g of waste plastics were fed to a batch pyrolysis reactor, and pyrolysis was performed at 500°C, thereby obtaining pyrolysis oil. The distribution of the mixture of hydrocarbon oils included in the pyrolysis oil is shown in Table 1.

[Table 1]

| Cut Name | Predictive carbon range | Boiling point (°C) | wt% |
|---|---|---|---|
| Naphtha | C7 to C9 | 80 to 150 | 22.1 |
| KEROSENE | C10 to C17 | 150 to 265 | 28.1 |
| LGO | C18 to C20 | 265 to 340 | 15.9 |
| VGO | C21 to | > 340 | 33.3 |
| Sum | - | - | 100 |

The pyrolysis oil was separated by boiling point through a distiller, and only C21+ VGO was selectively recovered, thereby preparing a pyrolysis oil feedstock. In the impurities in the recovered VGO, the contents of chlorine, nitrogen, and sulfur were 16 ppm, 265 ppm, and 23 ppm, respectively.

2) Preparation of Catalyst

70 g of Pseudo-boehmite was introduced into 500 g of water, 10 g of formic acid was introduced while stirring the mixture at room temperature, and then the mixture was maintained for 3 hours, thereby preparing alumina gel. Clay(Kaoline), ZSM-5, and MgO were introduced into a mixer so that an Mg/Al molar ratio was 25 and then stirring was performed for 10 minutes. The amount of MgO introduced was 5 wt% of the total weight of clay and ZSM-5. After the mixture of clay, ZSM-5, and MgO was stirred in the mixer to make the mixture into uniform small particles, the alumina gel was introduced, and then stirring was performed again with the mixer. 60 g of Ludox (AS 40) was introduced during stirring, and then a viscosity of the slurry was measured using a viscometer.

Thereafter, in the process of converting the viscosity of the slurry from sol to gel, the slurry was prepared into a spherical catalyst through spray drying, the spherical catalyst was dried in an oven at 120°C for 12 hours, and then the dried spherical catalyst was calcined at 550°C for 3 hours, thereby preparing a fluidized bed catalyst. The recovered catalyst was separated by screening only a catalyst having a particle size of 30 to 200 $\mu$m through sieving, the screened catalyst was subjected to ion-exchange with a 5% RE-metal solution at 60°C for 3 hours, the catalyst was dried, and then the dried catalyst was calcined at 550°C for 3 hours, thereby preparing a final catalyst.

3) Catalytic Cracking Process

The catalytic activity was evaluated by operating a davison circulating riser (DCR) reaction system for 120 minutes. A cracking reaction and a catalyst regeneration reaction in a regenerator were performed at 530°C and 730°C, respectively, and the reaction was performed while a stripper where a cracking reaction occurred was maintained at 527°C. A feedstock and steam were introduced into a reaction unit at 850 g/h and 80 g/h, respectively, $N_2$ was introduced at a total rate of 220 lps by introducing $N_2$ into a stripper at 180 lps and into a reactor at 40 lps, and the catalyst separated in a cyclone was introduced into the regenerator to be regenerated. Air was introduced at a rate of 900 lps for regeneration of the catalyst in the regenerator. The operation was performed under a condition in which a Cat/Oil ratio, which was a ratio of the catalyst to the feedstock introduced, was set to 11 to 20. A product was separated into a gas and a liquid in a stabilizer, and then the recovered gas was analyzed through GC, and the liquid was analyzed through SIMDIST. Coke,

$CO_2$, and $H_2$ were analyzed by a $CO/CO_2$ analyzer.

**Example 2**

A process was performed under the same conditions as those of Example 1, except that a catalyst was prepared so that the Mg/Al molar ratio of the catalytic cracking catalyst was 50.

**Example 3**

A process was performed under the same conditions as those of Example 1, except that a catalyst was prepared using hydrotalcite ($Mg_6Al_2CO_3(OH)_{16} \cdot 4H_2O$) instead of MgO in the preparation of the catalyst.

**Example 4**

A process was performed under the same conditions as those of Example 1, except that a catalyst was prepared using $MgSiO_3$ instead of MgO.

**Example 5**

A process was performed under the same conditions as those of Example 1, except that a catalyst was prepared using CaO instead of MgO.

**Example 6**

A process was performed under the same conditions as those of Example 1, except that a catalyst was prepared using $K_2O$ instead of MgO.

**Example 7**

A process was performed under the same conditions as those of Example 1, except that a regenerator was not used and a regeneration process of the catalyst was not performed.

**Comparative Example 1**

A process was performed under the same conditions as those of Example 1, except that a catalyst was prepared without using MgO.

**Evaluation Examples**

A davison circulating riser (DCR) reaction system was operated for 120 minutes, and analysis was performed on the produced oil. A gas component oil was quantified through on-line gas chromatography (model name HP 6890N), and a liquid component oil was recovered in a storage tank and then quantified through SIMDIST.
The contents (ppm) of chlorine, nitrogen, and sulfur in the finally obtained light olefins were evaluated by measurement through IC and TNS analysis methods.
The analysis results are shown in Table 2.

[Table 2]

| | Example 1 | Example 2 | Example 3 | Example 4 | Example 5 | Example 6 | Example 7 (Generation process X) | Comparative Example 1 |
|---|---|---|---|---|---|---|---|---|
| Catalyst composition | MgO, zeolite, clay, binder | MgO, zeolite , clay, binder | $Mg_6Al_2CO_3$ $(OH)_{16}\cdot 4\ H_2O$, zeolite, clay, binder | $MgSiO_3$, zeolite , clay, binder | CaO, zeolite , clay, binder | $K_2O$, zeolite , clay, binder | MgO, zeolite, clay, binder | Zeolite, clay, binder |
| Mg/Al molar ratio | 25 | 50 | 25 | 25 | - | - | 25 | - |
| Coke (wt%) | 6.7 | 11.2 | 5.9 | 14.6 | 7.5 | 14.7 | 15.6 | 16.5 |
| LCO+Naptha+ C4- oil (wt%) | 87 | 76.2 | 86.7 | 72.3 | 81.8 | 72.1 | 65.6 | 60.8 |
| Light olefins (ethylene + propylene + butylene + butadiene) (wt%) | 45.7 | 26.1 | 29.7 | 12.2 | 39.6 | 11.2 | 10.1 | 8 |
| Chlorine in light olefins (ppm) | < 1 | 4 | 2 | 6 | 3 | 5 | 9 | 14 |

EP 4 379 020 A1

As shown in Table 2, it could be confirmed that, in Examples 1 to 7 in which the catalytic cracking catalyst containing a compound of an alkali metal or an alkaline earth metal and a zeolite was used, the cracking efficiency, the light olefin conversion yield, and the chlorine reduction effect were excellent compared to those in Comparative Example 1.

Specifically, it could be confirmed that, in Example 1, as the catalytic cracking catalyst containing MgO and satisfying an Mg/Al molar ratio of 25 was used, the cracking efficiency, the light olefin conversion yield, and the chlorine reduction effect were most excellent.

It could be confirmed that, in Example 2, as the catalytic cracking catalyst satisfying an Mg/Al molar ratio of 50 was used, the cracking efficiency, the light olefin conversion yield, and the chlorine reduction effect were slightly deteriorated than those in Example 1, but were excellent compared to those in Comparative Example 1.

It could be confirmed that, in Example 3, as the catalytic cracking catalyst containing hydrotalcite ($Mg_6Al_2CO_3(OH)_{16} \cdot 4H_2O$) rather than MgO was used, the coke reduction effect was excellent compared to that in Example 1.

It could be confirmed that, in Example 4, as the catalytic cracking catalyst containing $MgSiO_3$ rather than MgO was used, the cracking efficiency, the light olefin conversion yield, and the chlorine reduction effect were slightly deteriorated than those in Example 1, but were excellent compared to those in Comparative Example 1.

It could be confirmed that, in Example 5, as the catalytic cracking catalyst containing CaO rather than MgO was used, the cracking efficiency and the light olefin conversion yield were second only to those in Example 1.

It could be confirmed that, in Example 6, as the catalytic cracking catalyst containing $K_2O$ rather than MgO was used, the cracking efficiency, the light olefin conversion yield, and the chlorine reduction effect were slightly deteriorated than those in Example 1, but were excellent compared to those in Comparative Example 1.

It could be confirmed that, in Example 7, as the regeneration process of the catalytic cracking catalyst was omitted, the cracking efficiency, the light olefin conversion yield, and the chlorine reduction effect were slightly deteriorated than those in Example 1, but were excellent compared to those in Comparative Example 1.

It could be confirmed that, in Comparative Example 1, as the catalytic cracking catalyst that did not contain a compound of an alkali metal or an alkaline earth metal was used, the cracking efficiency, the light olefin conversion yield, and the chlorine reduction effect were the lowest.

As set forth above, light olefins may be obtained from waste plastic pyrolysis oil with a high yield by the method according to the present invention.

The method according to the present invention may implement a conversion yield of 10% or more, specifically, 25% or more, and more specifically, 40% or more.

The method according to the present invention may stably perform a light olefin conversion process for a long time by preventing deactivation of the catalytic cracking catalyst due to impurities contained in the waste plastic pyrolysis oil using a fluidized bed reactor.

The method according to the present invention may minimize the content of impurities such as chlorine or nitrogen in the converted light olefins to several ppm.

**Claims**

1. A method for converting waste plastic pyrolysis oil into light olefins, the method comprising:

   (1) putting waste plastic pyrolysis oil into a reactor;
   (2) allowing the waste plastic pyrolysis oil to react in the reactor in the presence of a catalytic cracking catalyst containing a compound of an alkali metal or an alkaline earth metal and a zeolite; and
   (3) recovering light olefins by separating the catalytic cracking catalyst and oil from a product obtained in the step (2).

2. The method of claim 1, wherein the compound of an alkali metal or an alkaline earth metal includes an oxide, a hydroxide, a carbonate, a silicate, a sulfate, an acetate, a nitride, or an alkoxide.

3. The method of claim 1 or 2, wherein the compound of an alkali metal or an alkaline earth metal includes magnesium oxide.

4. The method of any one of claims 1 to 3, wherein the catalytic cracking catalyst satisfies an Mg/Al molar ratio of 10 to 40.

5. The method of any one of claims 1 to 4, wherein the zeolite includes ZSM-5, ZSM-11, Y-zeolite, ferrierite, mordenite, MCM-22, SUZ-4, or L-type zeolite.

6. The method of any one of claims 1 to 5, wherein the catalytic cracking catalyst further contains clay and a binder.

7. The method of claim 6, wherein the catalytic cracking catalyst contains 1 to 10 wt% of the compound of an alkali metal or an alkaline earth metal, 20 to 70 wt% of the zeolite, 10 to 60 wt% of the clay, and 10 to 50 wt% of the binder.

8. The method of any one of claims 1 to 7, wherein the catalytic cracking catalyst has an average particle size of 50 to 2,000 pm.

9. The method of any one of claims 1 to 8, wherein the waste plastic pyrolysis oil contains a vacuum gas oil (VGO) component having a boiling point of 340°C or higher at atmospheric pressure.

10. The method of any one of claims 1 to 9, wherein the step (2) is performed at a temperature of 400 to 600°C and a reaction pressure of 50 to 200 kPa.

11. The method of any one of claims 1 to 10, further comprising:

(3-1) recovering the catalytic cracking catalyst separated in the step (3), putting the recovered catalytic cracking catalyst into a regenerator, and then oxidizing the compound of an alkali metal or an alkaline earth metal; and
(3-2) circulating the catalytic cracking catalyst containing the oxidized compound of an alkali metal or an alkaline earth metal to the step (2) and re-feeding the catalytic cracking catalyst containing the oxidized compound of an alkali metal or an alkaline earth metal into the reactor.

12. The method of claim 11, wherein the step (3-1) is performed at 600 to 800°C in the presence of oxygen gas.

13. The method of claim 12, further comprising, before the step (3-1), stripping the catalytic cracking catalyst separated in the step (3).

14. A device for converting waste plastic pyrolysis oil into light olefins, the device comprising:

a fluidized bed reactor configured to receive waste plastic pyrolysis oil and to perform a catalytic cracking reaction in the presence of a catalytic cracking catalyst, the catalytic cracking catalyst containing a compound of an alkali metal or an alkaline earth metal and a zeolite;
a cyclone configured to receive a product from the fluidized bed reactor and to separate the product into the catalytic cracking catalyst and oil; and
a stabilizer configured to receive the oil from the cyclone and to separate the oil into a gas component and a liquid component.

15. The device of claim 14, further comprising:

a regenerator configured to receive the catalytic cracking catalyst from the cyclone and to oxidize the compound of an alkali metal or an alkaline earth metal,
a recirculation line configured to re-introduce the catalytic cracking catalyst containing the oxidized compound of an alkali metal or an alkaline earth metal into the fluidized bed reactor from the regenerator.

Europäisches Patentamt

European Patent Office

Office européen des brevets

# EUROPEAN SEARCH REPORT

Application Number

EP 23 21 1354

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | CN 114 656 318 A (QINGDAO TECHNOLOGY UNIV ET AL.) 24 June 2022 (2022-06-24) | 1-10 | INV. C10G1/00 |
| Y | * claims 1-9 * <br> * examples 1-6 * <br> * table 1 * | 8,11-15 | C10G11/05 C10G11/18 B01J29/40 B01J37/00 |
| X | CN 102 039 155 A (UNIV TONGJI) 4 May 2011 (2011-05-04) | 1-10 | |
| Y | * claims 1-4 * <br> * examples 1-3 * <br> * paragraphs [0010], [0015] - [0019] * | 8,11-15 | |
| X | US 2021/130262 A1 (WU XIANCHUN [US] ET AL) 6 May 2021 (2021-05-06) | 14,15 | |
| Y | * claims 1-20 * <br> * figures 1-4,26,27 * <br> * paragraphs [0600] - [0614] * | 8,11-15 | |

TECHNICAL FIELDS
SEARCHED        (IPC)

B01J
C10G

The present search report has been drawn up for all claims

| Place of search | Date of completion of the search | Examiner |
|---|---|---|
| The Hague | 25 March 2024 | Zuurdeeg, Boudewijn |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

.............................................................................................................

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 23 21 1354

This annex lists the patent family members relating to the patent documents cited in the above-mentioned European search report.
The members are as contained in the European Patent Office EDP file on
The European Patent Office is in no way liable for these particulars which are merely given for the purpose of information.

25-03-2024

| Patent document cited in search report | | Publication date | Patent family member(s) | | Publication date |
|---|---|---|---|---|---|
| CN 114656318 | A | 24-06-2022 | NONE | | |
| CN 102039155 | A | 04-05-2011 | NONE | | |
| US 2021130262 | A1 | 06-05-2021 | US | 2021130262 A1 | 06-05-2021 |
| | | | US | 2022234968 A1 | 28-07-2022 |

EPO FORM P0459

For more details about this annex : see Official Journal of the European Patent Office, No. 12/82